# EUROPEAN PATENT APPLICATION

(11) **EP 4 706 433 A1**
(43) Date of publication of application: **11.03.2026**
(21) Application number: 24797281.3
(22) Date of filing: 21.03.2024
(51) Int. Cl.: A24F 47/00, A61M 21/00

(54) **WEARABLE DEVICE AND SMOKING EFFECT PROVISION SYSTEM**

(30) Priority: 26.04.2023 KR 20230054524; 20.06.2023 KR 20230078628
(71) Applicant: KT&G Corporation, Daedeok-gu Daejeon 34337 (KR)
(72) Inventor: LEE, Won Kyeong, Guri-si Gyeonggi-do 11920 (KR); KIM, Min Kyu, Seoul 08211 (KR)
(74) Representative: AWA Sweden AB
(86) International application number: PCT/KR2024/003559
(87) International publication number: WO 2024/225619

(57) **Abstract**

A wearable device according to one embodiment can provide a smoking effect, and may comprise: a first stimulation member arranged at a location corresponding to a first area of a user, so as to provide electrical stimulation; and a second stimulation member arranged at a location corresponding to a second area of the user, so as to provide a sound signal, wherein the first stimulation member or the second stimulation member can provide a stimulation signal for inducing an arousal state in which a smoking effect can be implemented.

## Description

### TECHNICAL FIELD

Methods and apparatuses consistent with embodiments relate to a wearable device and a system for providing a smoking effect.

### BACKGROUND ART

Research on an apparatus for generating electroencephalogram (EEG) is in progress. For example, Korean Patent Publication No. 10-2011-0064706 discloses an apparatus and method for generating EEG.

### DISCLOSURE OF THE INVENTION

### TECHNICAL GOALS

Embodiments provide a smoking effect to a user without an act of smoking.

Embodiments effectively provide a smoking effect through stimulation of the brain of a user.

Embodiments provide a method of performing brain stimulation appropriate for an environment by selectively and/or simultaneously performing brain stimulation by electrical stimulation or brain stimulation by sound stimulation.

Embodiments provide an effective control of the degree of brain stimulation based on feedback from a user.

### TECHNICAL SOLUTIONS

According to an aspect of an embodiment, there is provided a wearable device for providing a smoking effect. The wearable device includes a first stimulus member disposed in a position corresponding to a first region of a user and configured to provide electrical stimulation, a second stimulus member disposed in a position corresponding to a second region of the user and configured to provide sound stimulation, wherein the first stimulus member or the second stimulus member is configured to provide a stimulus signal that induces an alertness state capable of implementing a smoking effect.

The wearable device may further include a link that connects the first stimulus member to the second stimulus member, wherein the link may be configured to be hooked to an auricle of the user.

The first region may be a rear of an auricle, and the second region may be an auricle or a surface of an external auditory canal.

The wearable device may further include a controller configured to control at least one of the first stimulus member or the second stimulus member.

The controller may further be configured to control at least one of the first stimulus member or the second stimulus member so that the first stimulus member or the second stimulus member provides a brain stimulus signal that increases power spectral density (PSD) of electroencephalogram (EEG) of the user in a frequency region of 10 Hz or higher.

The controller may further be configured to control the first stimulus member so that the first stimulus member provides electrical stimulation that causes PSD of EEG of the user to increase by 0.1 to 1.1 dB in a frequency range of 12 Hz to 25 Hz, or to control the second stimulus member so that the second stimulus member provides sound stimulation that causes the PSD of the EEG of the user to increase by 0.1 to 1.1 dB in the frequency range of 12 Hz to 25 Hz.

The controller may further be configured to cause the second stimulus member to form a binaural beat that provides beta waves.

The controller may further be configured to determine, based on a surrounding environment of the user or state information of the user, whether to perform one of brain stimulation by the first stimulus member, brain stimulation by the second stimulus member, or brain stimulation by both of the first stimulus member and the second stimulus member.

The wearable device may further include a verification member configured to verify a smoking effect, wherein the controller may further be configured to determine a degree of activation of at least one of the first stimulus member or the second stimulus member based on a smoking indicator signal transmitted from the verification member.

The wearable device may further include a verification member configured to verify a smoking effect, wherein the verification member may be capable of measuring a heart rate variability (HRV).

According to an aspect of an embodiment, there is provided a system for providing a smoking effect. The system includes a first wearable device that is worn on a right ear of a user and a second wearable device that is worn on a left ear of the user, wherein the first wearable device includes a first stimulus member disposed at a rear of the right ear and configured to provide electrical stimulation and a second stimulus member disposed on an auricle or a surface of an external auditory canal of the right ear and configured to provide sound stimulation, wherein the second wearable device includes a third stimulus disposed at the rear of the left ear and configured to provide electrical stimulation and a fourth stimulus member disposed on an auricle or a surface of an external auditory canal of the left ear and configured to provide sound stimulation, and wherein at least one of the first stimulus member, the second stimulus member, the third stimulus member, or the fourth stimulus member is configured to provide a brain stimulus signal capable of implementing a smoking effect.

The system may further include a controller configured to control at least one of the first stimulus member, the second stimulus member, the third stimulus member, or the fourth stimulus member, wherein the controller may further be configured to control at least one of the first stimulus member, the second stimulus member, the third stimulus member, or the fourth stimulus member so that at least one of the first stimulus member, the second stimulus member, the third stimulus member, or the fourth stimulus member provides at least one of electrical stimulation or sound stimulation that causes power spectral density (PSD) of electroencephalogram (EEG) of the user to increase by 0.1 to 1.1 dB in a frequency range of 12 to 25 Hz.

The controller may further be configured to cause a difference between a frequency of sound stimulation of the second stimulus member and a frequency of sound stimulation of the fourth stimulus member to be from 12 to 25 Hz.

The controller may further be configured to cause the second stimulus member and the fourth stimulus member to form a binaural beat that provides beta waves.

### EFFECTS OF THE INVENTION

According to embodiments, a smoking effect may be provided to a user without an act of smoking.

According to embodiments, a smoking effect may effectively be provided through stimulation of the brain of a user.

According to embodiments, brain stimulation by electrical stimulation or brain stimulation by sound stimulation may be selectively and/or simultaneously performed and thus, brain stimulation appropriate for a use environment may be performed.

According to embodiments, the degree of brain stimulation may effectively be controlled based on feedback from a user.

The effects of a wearable device and a system for providing a smoking effect are not limited to the above-mentioned effects, and other unmentioned effects may be clearly understood from the following description by one of ordinary skill in the art.

### BRIEF DESCRIPTION OF THE DRAWINGS

The above and/or other aspects will be more apparent by describing certain embodiments with reference to the accompanying drawings, in which:
FIG. 1 is a diagram illustrating a wearable device according to an embodiment;
FIG. 2 is a diagram illustrating a wearing state of a wearable device according to an embodiment;
FIG. 3 is a diagram illustrating electroencephalogram (EEG) of a user before smoking and after smoking;
FIG. 4 is a diagram illustrating EEG of a user before and after stimulation by a wearable device according to an embodiment;
FIGS. 5A to 5E are diagrams illustrating indicators of a heart rate variability (HRV) of a user before smoking and after smoking;
FIGS. 6A and 6B are diagrams illustrating an autonomic balance among the HRV indicators of a user before and after stimulation by a wearable device or a system for providing a smoking effect, according to an embodiment; and
FIG. 7 is a diagram illustrating a system for providing a smoking effect according to an embodiment.

### BEST MODE FOR CARRYING OUT THE INVENTION

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, expressions "at least one of a, b, or c" and "at least one of a, b, and c" should be construed as referring to "a," "b," "c," "a and b," "a and c," "b and c," or "a, b, and c."

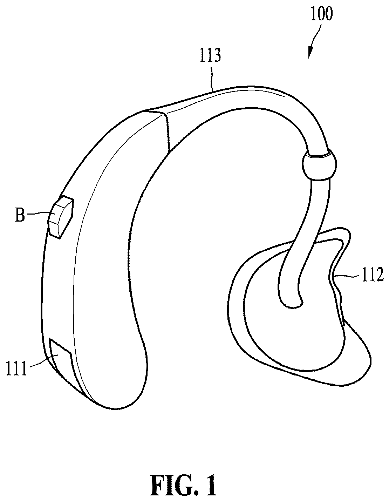

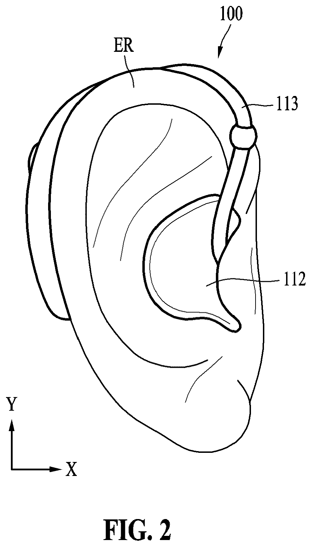

FIG. 1 is a diagram illustrating a wearable device 100 according to an embodiment, and FIG. 2 is a diagram schematically illustrating a use state of the wearable device 100 according to an embodiment.

Referring to FIGS. 1 and 2, the wearable device 100 may provide a smoking effect, and the wearable device 100 may include a first stimulus member 111, a second stimulus member 112, a link 113, and a controller.

The first stimulus member 111 may be disposed on a position corresponding to a first region of a user to provide electrical stimulation. The first region may be a posterior (e.g., the side in the -X direction of FIG. 2) of an auricle of an ear (e.g., a right ear ER of FIG. 2) of the user.

The first stimulus member 111 may include an electrode that performs electrical stimulation. The electrode may be connected to a power supply and the controller accommodated in the wearable device 100.

The second stimulus member 112 may be disposition in a position corresponding to a second region of the user to provide sound stimulation. The second region may be the auricle or a surface of an external auditory canal of the ear of the user.

For example, the second stimulus member 112 may include an earpiece. The earpiece may be a device capable of receiving sound data from the wearable device 100 and may include a device that may be worn on the ear of the user and that may receive sound data, such as an earbud, an earphone, hearing electronics, and the like.

The link 113 may connect the first stimulus member 111 to the second stimulus member 112 and may be configured to be hooked to the auricle of the user.

The link 113 of the wearable device 100 may be worn on the body of the user (e.g., the right ear ER of FIG. 2) with the first stimulus member 111 and the second stimulus member 112 in contact with the first region and the second region of the user, respectively. The link 113 may be made of metal or plastic material. A portion of the link 113 (e.g., an edge) may be made of metal material and the other portion of the link 113 may be made of plastic material.

The wearable device 100 may have a button B on an outside, and operations of the wearable device 100 may be controlled through the button B. For example, when the operation of the wearable device 100 is initiated, brain stimulation may be performed for a preset period of time. After the preset period of time, the brain stimulation may be stopped and a notification may be sent to the user indicating the brain stimulation has stopped.

An internal space of the wearable device 100 may additionally accommodate a memory, a communication unit, and the like.

For example, the memory may be hardware for storing various pieces of data that are processed in the wearable device 100 and may store data that has been processed by the controller and data to be processed by the controller. The memory may include at least one type of storage medium of flash memory-type memory, hard disk-type memory, multimedia card micro-type memory, card-type memory (e.g., a secure digital (SD) or extreme digital (XD) memory), random-access memory (RAM), static random-access memory (SRAM), read-only memory (ROM), electrically erasable programmable read-only memory (EEPROM), programmable read-only memory (PROM), magnetic memory, a magnetic disk, or an optical disk. The memory may store an operating time, a maximum use time, and a current use time of the wearable device 100, and data on a smoking pattern of the user.

For example, the communication unit may include a short-range wireless communication unit and a wireless communication unit. The short-range wireless communication unit may include a Bluetooth communication unit, a Bluetooth Low Energy (BLE) communication unit, a near field communication unit, a wireless local area network (WLAN) (e.g., wireless fidelity (Wi-Fi)) communication unit, a ZigBee communication unit, an infrared data association (IrDA) communication unit, a Wi-Fi direct (WFD) communication unit, an ultra-wideband (UWB) communication unit, an Ant+ communication unit, and the like. However, embodiments are not limited thereto. The wireless communication unit may include a cellular network communication unit, an Internet communication unit, a computer network (e.g., a local area network (LAN) or a wide-area network (WAN)) communication unit, and the like. However, embodiments are not limited thereto.

The controller of the wearable device 100 may control the first stimulus member 111 and/or the second stimulus member 112 so that the first stimulus member 111 and/or the second stimulus member 112 may generate a stimulus signal that induces a state of alertness that may implement a smoking effect. For example, the controller may be accommodated in the internal space of the wearable device 100 and connected to the first stimulus member 111 and/or the second stimulus member 112. In another example, the controller may be accommodated in another electronic device (e.g., a mobile communication terminal) outside of the wearable device 100 and may provide a control signal to the first stimulus member 111 and/or the second stimulus member 112 through the communication unit.

The first stimulus member 111 and/or the second stimulus member 112 may generate the stimulus signal that may stimulate the brain of the user. For example, the stimulus signal may include electrical stimulation and/or sound stimulation.

The controller of wearable device 100 may control the first stimulus member 111 and/or the second stimulus member 112 to provide a brain stimulus signal that may increase a beta waves region of electroencephalogram (EEG) of the user. The first stimulus member 111 and/or the second stimulus member 112 may provide the stimulus signal (e.g., a stimulus signal that may increase beta waves of the EEG of the user) that induces a state of alertness that may implement a smoking effect.

A frequency range of delta waves may be from 1 Hz to 4 Hz, and delta waves are associated with an involuntary physical activity, such as the heartbeat and digestion. When power associated with delta waves is increased, a state of rest and sleep may be induced in a user O and a situation such as relaxation may be implemented. On the contrary, when the power associated with delta waves is decreased, a state of alertness may be induced. A frequency range of theta waves may be from 4 Hz to 8 Hz, and theta waves may mainly occur during meditation or when a hippocampus is activated. The hippocampus may be a region that controls a memory. When power associated with theta waves is increased, a state of inspiration, insight, or connecting pieces of memory may be induced. On the contrary, when the power associated with theta waves is decreased, a state of anxiety, stress, and low emotional self-awareness may be induced. A frequency range of beta waves may be from 12 Hz to 25 Hz, and since beta waves normally occur during alertness, an increase in beta waves may indicate alertness. When power associated with beta waves is increased, a state of alertness such as a situation of cognitive thought activity may be induced. On the contrary, when the power associated with beta waves are decreased, a state of relaxation may be induced.

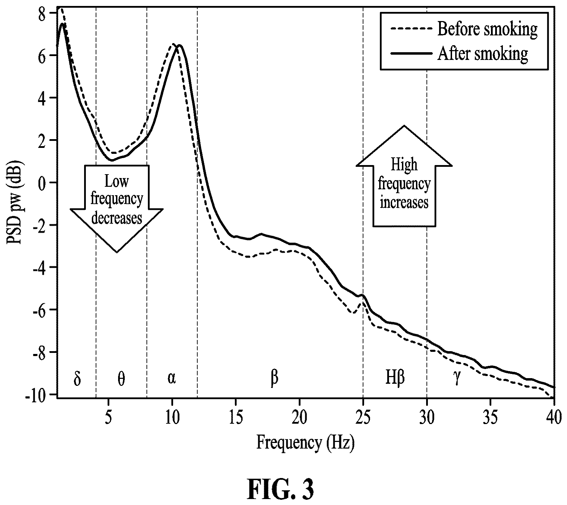

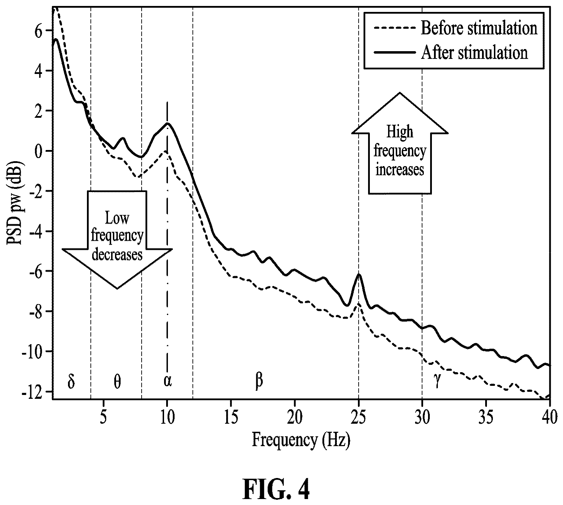

FIG. 3 is a diagram illustrating EEG of a user before and after smoking a cigarette or an electronic cigarette (e-cigarette), and FIG. 4 is a diagram illustrating EEG of a user before and after stimulation by the wearable device 100 or a system for providing a smoking effect.

Referring to FIG. 3, the graph in FIG. 3 shows a measurement of the EEG of a subject before and after smoking a cigarette or an e-cigarette, and an electrophysiological method of recording electrical activity of a brain through an electrode was used. The detailed method of the experiment is as follows. EEG sensors (i.e., sensors for measuring EEG) were attached to a plurality of positions on the heads of subjects. The subjects were divided into two groups, a cigarette group and an e-cigarette group, and EEG was measured before and after smoking in each of the cases. Results of analysis of power spectral density (PSD) before and after smoking are shown in the graph of FIG. 3. Referring to FIG. 3, as a result of analysis of pieces of power of all bands, it is confirmed that the low-frequency (e.g., delta waves and theta waves) range tends to decrease and the high-frequency (e.g., beta waves) range tends to increase after smoking. For example, it is confirmed that PSD of the EEG of the user in a frequency range of 10 Hz or higher tends to increase in general.

Referring to FIG. 4, a same or similar result as actual smoking shown in FIG. 3 was implemented using the wearable device 100 by configuring the first stimulus member 111 and/or the second stimulus member 112 to perform brain stimulation such as electrical stimulation and/or sound stimulation so that the low-frequency range of the EEG of a user may decrease and the high-frequency range of the EEG of the user may increase after the brain stimulation.

As shown in FIG. 4, electrical stimulation and/or sound stimulation by the wearable device 100 may implement an actual smoking effect by changing the power of the EEG band (e.g., increasing the power of beta waves).

In addition, electrical stimulation from the first stimulus member 111 may be transcranial alternating current stimulation (tACS), and the tACS may affect a change in periodic excitability of a neuron in a cerebral cortex with a maximum current within 2 mA of a sine waveform at various frequencies and may induce a change in a brain function such as cognition and memory. An applied frequency may be 10 Hz, and a stimulation time may be 10 to 30 minutes.

Sound stimulation from the second stimulus member 120 may induce beta waves stimulation of the EEG of the user by utilizing a binaural beat. The binaural beat may send different frequency signals to the ears of the user so that the brain may perceive a difference between the two frequencies. Here, the difference between the two frequencies may be 12 Hz to 25 Hz, which corresponds to beta waves.

The controller of the wearable device 100 may control the first stimulus member 111 and/or the second stimulus member 112 so that the first stimulus member 111 and/or the second stimulus member 112 may provide a brain stimulus signal that may increase the frequency of an EEG region of the user corresponding to 10 Hz or higher.

The controller of the wearable device 100 may control the first stimulus member 111 so that the first stimulus member 111 may provide electrical stimulation that may cause the PSD of the EEG of the user to increase by 0.1 to 1.1 dB in a frequency range of 12 Hz to 25 Hz.

In addition, the controller of the wearable device 100 may control the first stimulus member 111 so that the first stimulus member 111 may provide electrical stimulation that may cause the PSD of the EEG of the user to decrease by 0.3 to 0.9 dB in a frequency range of 1 Hz to 4 Hz. Furthermore, the controller of the wearable device 100 may control the first stimulus member 111 so that the first stimulus member 111 may provide electrical stimulation that may cause the PSD of the EEG of the user to decrease by 0.2 to 0.5 dB in a frequency range of 4 Hz to 8 Hz.

The controller of the wearable device 100 may control the second stimulus member 112 so that the second stimulus member 112 may provide sound stimulation that may cause the PSD of the EEG of the user to increase by 0.1 to 1.1 dB in a frequency range of 12 Hz to 25 Hz.

The controller of the wearable device 100 may simultaneously control the first stimulus member 111 and the second stimulus member 112 so that the first stimulus member 111 and the second stimulus member 112 may cause the PSD of the EEG of the user to increase by 0.1 to 1.1 dB in a frequency range of 12 Hz to 25 Hz.

The controller of the wearable device 100 may determine whether to perform brain stimulation using the first stimulus member 111, perform brain stimulation using the second stimulus member 112, or perform brain stimulation using both of the first stimulus member 111 and the second stimulus member 112, depending on a surrounding environment or a state of the user.

For example, when a sound noise around the wearable device 100 is greater than or equal to a set value, the controller of the wearable device 100 may provide a smoking effect to the user by performing brain stimulation by electrical stimulation through the first stimulus member 111 instead of the second stimulus member 112.

In another example, when an area around the wearable device 100 is in a state of over-humidity such as in a rainy season or when sweat is detected on a skin of the user above the set value, the controller of the wearable device 100 may determine that the condition is unfavorable to electrical stimulation and may provide a smoking effect to the user by performing brain stimulation by sound stimulation through the second stimulus member 112.

In another example, when an increased smoking effect needs to be provided in a short period of time, the controller of the wearable device 100 may simultaneously operate the first stimulus member 111 and the second stimulus member 112 to perform rapid and intense brain stimulation.

The wearable device 100 may further include a verification member that verifies a smoking effect. The verification member may measure a heart rate variability (HRV).

An HRV may refer to a comprehensive combination of indicators, such as a heart rate (HR), a mean interval between electrocardiogram (ECG) peaks (mean R-R interval), a standard deviation of normal to normal R-R intervals (SDNN), which indicates stress resistance (physiological resilience), a root mean square of successive heartbeat interval differences (RMSSD), which is a parasympathetic index that is involved in electrical stability of the heart, low frequency (LF), which is a relatively low-frequency component and an indicator of a sympathetic nervous activity, high frequency (HF), which is a relatively high-frequency component and related to a respiratory activity, and the like. A healthier person may have a complex HRV. Here, an increase in an HR may indicate an excitement and an increase in load (physical activity), a decrease in an SDNN may indicate a monotonous HR fluctuation signal, a decrease in an ability to cope with stress, and a decrease in overall health, a decrease in an RMSSD may indicate a heart abnormality, and an increase in an LF may indicate a decrease in an HRV.

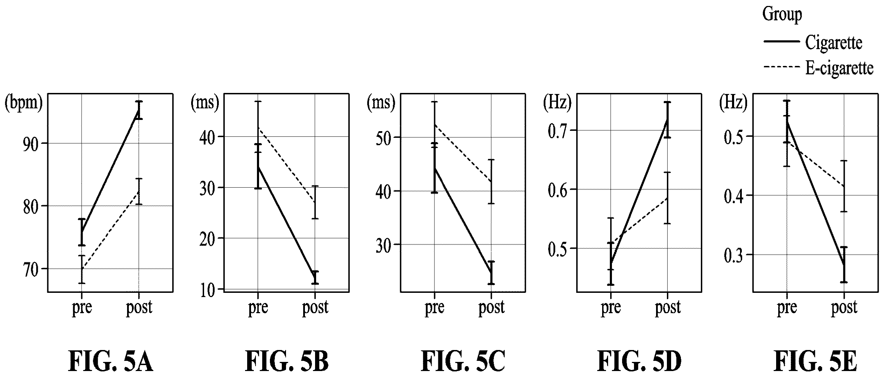

FIGS. 5A to 5E are diagrams illustrating indicators of an HRV of a user before and after smoking a cigarette or an e-cigarette. In the case of an e-cigarette, effects of an e-cigarette containing nicotine on ECG indicators before and after smoking are shown. FIG. 5A illustrates a mean HR, FIG. 5B illustrates an RMSSD, FIG. 5C illustrates an SDNN, FIG. 5D illustrates an LF band, and FIG. 5E illustrates an HF band. Referring to FIGS. 5A to 5E, the mean HR and the LF band increased, while the SDNN, the RMSSD, and the HF band decreased. This may indicate a physiological and psychological state similar to an alertness in response to stimulation.

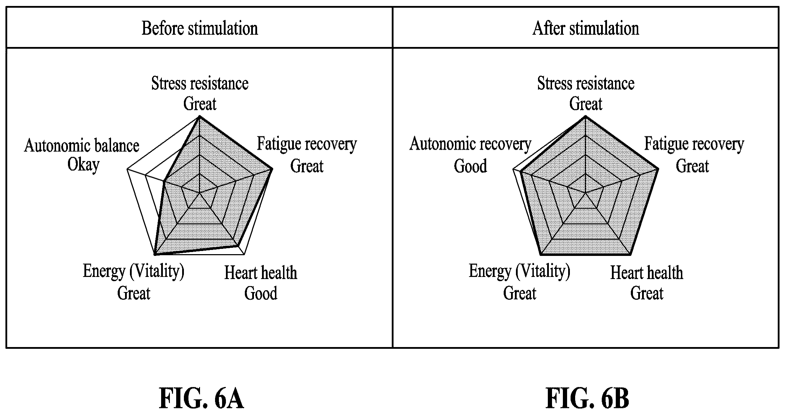

FIGS. 6A and 6B are diagrams illustrating a change in HRV indicators (e.g., an autonomic balance) caused by electrical stimulation through the wearable device 100. FIG. 6A illustrates the autonomic balance before the electrical stimulation and FIG. 6B illustrates the autonomic balance after the electrical stimulation. As shown in FIGS. 6A and 6B, it may be confirmed that the autonomic balance improves after the electrical stimulation compared to before the electrical stimulation. This may indicate a physiological and psychological state similar to an alertness in response to stimulation.

The alertness effect after stimulation (providing a smoking effect) by the wearable device 100 may be confirmed through a verification member. For example, the verification member may utilize an SDNN or an RMSSD among the HRV indicators to confirm a decrease in the SDNN or the RMSSD and/or a degree of decrease in the SDNN and the RMSSD before and after the stimulation and may thus verify whether a sufficient alertness effect (i.e., a smoking effect) has been achieved.

The controller of the wearable device 100 may determine a degree of activation of the first stimulus member 111 and/or the second stimulus member 112 based on a smoking indicator (e.g., the HRV indicators) signal transmitted from the verification member. For example, when a degree of decrease in the SDNN or the RMSSD confirmed by the verification member is sufficient enough to be equivalent to a prestored degree of decrease in the SDNN or the RMSSD in actual smoking, the controller may maintain a control state of the first stimulus member 111 and/or the second stimulus member 112. On the contrary, when a degree of decrease in the SDNN or the RMSSD confirmed by the verification member is not sufficient enough to be equivalent to the prestored degree of decrease in the SDNN or the RMSSD in actual smoking, the controller may increase or decrease a stimulation intensity of the first stimulus member 111 and/or the second stimulus member 112.

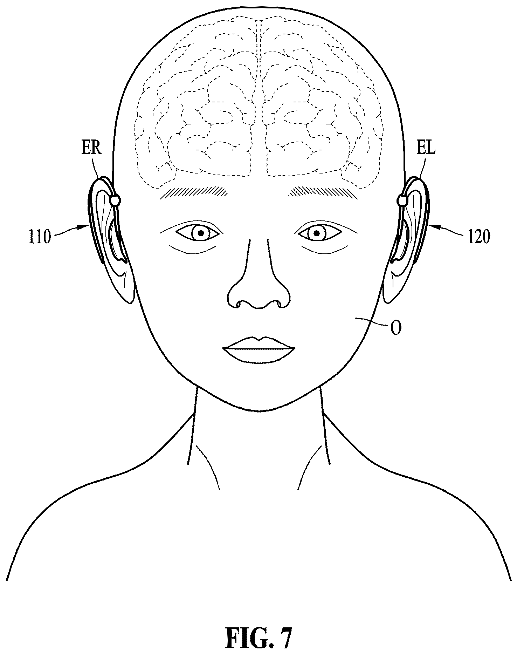

FIG. 7 is a diagram illustrating a system for providing a smoking effect. The system for providing a smoking effect may include a first wearable device 110 (e.g., the wearable device 100 of FIG. 1) that may be worn on the right ear ER of the user O and a second wearable device 120 that may be worn on a left ear EL of the user O.

The first wearable device 110 may include a first stimulus member (e.g., the first stimulus member 111 of FIG. 1) disposed at the rear of the right ear ER to provide electrical stimulation and a second stimulus member (e.g., the second stimulus member 112 of FIG. 112) disposed on an auricle or a surface of an external auditory canal of the right ear ER to provide first sound stimulation.

The second wearable device 120 may include a third stimulus member disposed at the rear of the left ear EL to provide electrical stimulation and a fourth stimulation member disposed on an auricle or a surface of an external auditory canal of the left ear EL to provide second sound stimulation. Since the third stimulus member may be configured to be identical or similar to the first stimulus member 111 and the fourth stimulus member may be configured to be identical or similar to the second stimulus member 112, a detailed description of the third stimulus member and the fourth stimulus member is omitted herein for simplicity. At least one of the first stimulus member 111, the second stimulus member 112, the third stimulus member, or the fourth stimulus member may provide a stimulus signal that may implement a smoking effect.

For example, a controller of the first wearable device 110 may control the first stimulus member 111 and the second stimulus member 112, and a controller of the second wearable device 120 may control the third stimulus member and the fourth stimulus member. In this case, the controller of the first wearable device 110 and the controller of the second wearable device 120 may be synchronized with each other. Alternatively, a controller may be provided on one of the first wearable device 110 or the second wearable device 120, wherein the controller may control all of the first stimulus member 111, the second stimulus member 112, the third stimulus member, and the fourth stimulus member.

The first stimulus member 111 and the third stimulus member may provide electrical stimulation to the rear of the ear of the user. For example, the first stimulus member 111 may include an anode (+), and the third stimulus member may include a cathode (-). The controller may cause an electric current to flow from the anode of the first stimulus member 111 to the cathode of the third stimulus member and to flow from the rear of the right ear ER of the user O through the brain of the user O to the rear of the left ear EL of the user O. Here, an excitability of neurons in the cortex of the brain adjacent to the first stimulus member 111 may increase and an excitability of neurons in the cortex of the brain adjacent to the third stimulus member may decrease so that an alertness effect of the user may be maximized.

The controller may provide an alertness effect to the user O by causing the second stimulus member 112 of the first wearable device 110 and the fourth stimulus member of the second wearable device 120 to form a binaural beat that provides beta waves. For example, the controller may adjust EEG according to a frequency difference of music or white noise injected into both ears of the user and create beta waves that may provide an alertness effect to the user O.

For example, the controller may cause a difference between a frequency of sound stimulation of the second stimulus member 112 of the first wearable device 110 and a frequency of sound stimulation of the fourth stimulus member of the second wearable device 120 to be from 12 to 25 Hz.

According to the wearable devices 110 and 120 and the system 10 for providing a smoking effect, a smoking effect may be provided to the user O without an act of smoking. In this case, a smoking effect may effectively be provided through stimulation of the brain of the user O. The wearable devices 110 and 120 and the system 10 for providing a smoking effect may selectively and/or simultaneously perform brain stimulation by electrical stimulation or brain stimulation by sound stimulation to allow brain stimulation optimized for a use environment. As the wearable devices 110 and 120 and the system 10 for providing a smoking effect may adjust a degree of stimulus activation based on feedback (e.g., the HRV of the user) from the user, the degree of brain stimulation may effectively be controlled.

The descriptions of the above-described embodiments are only examples, and it will be understood by one of ordinary skill in the art that various changes and equivalents may be made therefrom. Therefore, the scope of the disclosure should be defined by the appended claims, and all differences within the scope equivalent to those described in the claims will be construed as being included in the scope of protection defined by the claims.

## Claims

1. A wearable device for providing a smoking effect, the wearable device comprising:
a first stimulus member disposed in a position corresponding to a first region of a user and configured to provide electrical stimulation; and
a second stimulus member disposed in a position corresponding to a second region of the user and configured to provide sound stimulation,
wherein the first stimulus member or the second stimulus member is configured to provide a stimulus signal that induces an alertness state capable of implementing a smoking effect.

2. The wearable device of claim 1, further comprising:
a link that connects the first stimulus member to the second stimulus member,
wherein the link is configured to be hooked to an auricle of the user.

3. The wearable device of claim 1, wherein
the first region is a rear of an auricle, and
the second region is an auricle or a surface of an external auditory canal.

4. The wearable device of claim 1, further comprising:
a controller configured to control at least one of the first stimulus member or the second stimulus member.

5. The wearable device of claim 4, wherein the controller is further configured to control at least one of the first stimulus member or the second stimulus member so that the first stimulus member or the second stimulus member provides a brain stimulus signal that increases power spectral density (PSD) of electroencephalogram (EEG) of the user in a frequency region of 10 Hz or higher.

6. The wearable device of claim 4, wherein
the controller is further configured to control the first stimulus member so that the first stimulus member provides electrical stimulation that causes PSD of EEG of the user to increase by 0.1 to 1.1 dB in a frequency range of 12 Hz to 25 Hz, or
the controller is further configured to control the second stimulus member so that the second stimulus member provides sound stimulation that causes the PSD of the EEG of the user to increase by 0.1 to 1.1 dB in the frequency range of 12 Hz to 25 Hz.

7. The wearable device of claim 4, wherein the controller is further configured to cause the second stimulus member to form a binaural beat that provides beta waves.

8. The wearable device of claim 4, wherein the controller is further configured to determine, based on a surrounding environment of the user or state information of the user, whether to perform one of:
brain stimulation by the first stimulus member;
brain stimulation by the second stimulus member; or
brain stimulation by both of the first stimulus member and the second stimulus member.

9. The wearable device of claim 4, further comprising:
a verification member configured to verify a smoking effect,
wherein the controller is further configured to determine a degree of activation of at least one of the first stimulus member or the second stimulus member based on a smoking indicator signal transmitted from the verification member.

10. The wearable device of claim 1, further comprising:
a verification member configured to verify a smoking effect,
wherein the verification member is capable of measuring a heart rate variability (HRV).

11. A system for providing a smoking effect, the system comprising:
a first wearable device that is worn on a right ear of a user; and
a second wearable device that is worn on a left ear of the user,
wherein the first wearable device comprises:
a first stimulus member disposed at a rear of the right ear and configured to provide electrical stimulation; and
a second stimulus member disposed on an auricle or a surface of an external auditory canal of the right ear and configured to provide sound stimulation,
wherein the second wearable device comprises:
a third stimulus disposed at the rear of the left ear and configured to provide electrical stimulation; and
a fourth stimulus member disposed on an auricle or a surface of an external auditory canal of the left ear and configured to provide sound stimulation, and
wherein at least one of the first stimulus member, the second stimulus member, the third stimulus member, or the fourth stimulus member is configured to provide a brain stimulus signal capable of implementing a smoking effect.

12. The system of claim 11, further comprising:
a controller configured to control at least one of the first stimulus member, the second stimulus member, the third stimulus member, or the fourth stimulus member,
wherein the controller is further configured to control at least one of the first stimulus member, the second stimulus member, the third stimulus member, or the fourth stimulus member so that at least one of the first stimulus member, the second stimulus member, the third stimulus member, or the fourth stimulus member provides at least one of electrical stimulation or sound stimulation that causes power spectral density (PSD) of electroencephalogram (EEG) of the user to increase by 0.1 to 1.1 dB in a frequency range of 12 to 25 Hz.

13. The system of claim 12, wherein the controller is further configured to cause a difference between a frequency of sound stimulation of the second stimulus member and a frequency of sound stimulation of the fourth stimulus member to be from 12 to 25 Hz.

14. The system of claim 12, wherein the controller is further configured to cause the second stimulus member and the fourth stimulus member to form a binaural beat that provides beta waves.
